# EUROPEAN PATENT SPECIFICATION

(11) **EP 3 893 920 B1**
(45) Date of publication and mention of the grant of the patent: **18.09.2024**
(21) Application number: 19838946.2
(22) Date of filing: 10.12.2019
(51) Int. Cl.: A61K 38/40, A61K 31/726, A61P 17/00, A61P 17/02

(54) **COMPOSITION FOR THE TREATMENT OF SKIN LESIONS**
ZUSAMMENSETZUNG ZUR BEHANDLUNG VON HAUTLÄSIONEN
COMPOSITION POUR LE TRAITEMENT DE LÉSIONS CUTANÉES

(30) Priority: 11.12.2018 IT 201800010968
(43) Date of publication of application: 20.10.2021
(73) Proprietor: Universitá Degli Studi Di Salerno, 84084 Fisciano (IT)
(72) Inventor: PETRELLA, Antonello, 80127 Napoli (IT); PETRELLA, Francesco, 81031 Aversa (IT); BELVEDERE, Raffaella, 84010 San Marzano Sul Sarno (IT); BIZZARRO, Valentina, 84081 Baronissi (IT); PESSOLANO, Emanuela, 84031 Auletta (IT); PORTA, Amalia, 83042 Atripalda (IT); TOSCO, Alessandra, 84121 Salerno (IT)
(74) Representative: Valenza, Silvia
(86) International application number: PCT/IB2019/060595
(87) International publication number: WO 2020/121179

(56) References cited:
- WO-A1-2012/153301
- WO-A1-2012/153301
- US-A1- 2010 329 995
- US-A1- 2010 329 995
- AROSIO E FERRARI G SANTORO L GIANESE F COCCHERI S ET AL: "A placebo-controlled, double-blind study of mesoglycan in the treatment of chronic venous ulcers", EUROPEAN JOURNAL OF VASCULAR AND ENDOVASCULAR SURGERY, SAUNDERS, LONDON, GB, vol. 22, no. 4, 1 October 2001 (2001-10-01), pages 365 - 372, XP002693997, ISSN: 1078-5884, DOI: 10.1053/EJVS.2001.1478
- RAFFAELLA BELVEDERE ET AL: "Effects of Prisma Skin dermal regeneration device containing glycosaminoglycans on human keratinocytes and fibroblasts", CELL ADHESION & MIGRATION, 10 August 2017 (2017-08-10), United States, pages 168 - 16, XP055615631, Retrieved from the Internet <URL:https://www.tandfonline.com/doi/pdf/10.1080/19336918.2017.1340137?needAccess=true> [retrieved on 20190827], DOI: 10.1080/19336918.2017.1340137
- JULIA BENEDETTI: "Description of Skin Lesions -Dermatologic Disorders", MERCH MANUAL PROFESSIONAL VERSION, 1 January 2018 (2018-01-01), XP055615721, Retrieved from the Internet <URL:https://www.merckmanuals.com/professional/dermatologic-disorders/approach-to-the-dermatologic-patient/description-of-skin-lesions?query=skin%20lesions> [retrieved on 20190827]
- AROSIO E FERRARI G SANTORO L GIANESE F COCCHERI S ET AL: "A placebo-controlled, double-blind study of mesoglycan in the treatment of chronic venous ulcers", EUROPEAN JOURNAL OF VASCULAR AND ENDOVASCULAR SURGERY, SAUNDERS, LONDON, GB, vol. 22, no. 4, 1 October 2001 (2001-10-01), pages 365 - 372, XP002693997, ISSN: 1078-5884, DOI: 10.1053/EJVS.2001.1478
- RAFFAELLA BELVEDERE ET AL: "Effects of Prisma Skin dermal regeneration device containing glycosaminoglycans on human keratinocytes and fibroblasts", CELL ADHESION & MIGRATION, 10 August 2017 (2017-08-10), United States, pages 168 - 16, XP055615631, Retrieved from the Internet <URL:https://www.tandfonline.com/doi/pdf/10.1080/19336918.2017.1340137?needAccess=true> [retrieved on 20190827], DOI: 10.1080/19336918.2017.1340137
- JULIA BENEDETTI: "Description of Skin Lesions -Dermatologic Disorders", MERCH MANUAL PROFESSIONAL VERSION, 1 January 2018 (2018-01-01), XP055615721, Retrieved from the Internet <URL:https://www.merckmanuals.com/professional/dermatologic-disorders/approach-to-the-dermatologic-patient/description-of-skin-lesions?query=skin%20lesions> [retrieved on 20190827]

## Description

### FIELD OF THE INVENTION

The present invention relates to the field of pharmaceutical or cosmetic compositions comprising the association of lactoferrin and mesoglycan.

### BACKGROUND

Chronic skin lesions (or skin ulcers) represent one of the most expensive health services: about 3% of health expenditure is used worldwide to treat this condition. In Italy, chronic skin lesions affect over 2 million people, of which 30,000 are children. More than 10 million are in Europe. 80 million people worldwide are estimated to be affected. Skin ulcers are wounds without a spontaneous tendency to cicatrisation, they are generally the consequence of vascular pathologies or diabetes. The most common forms are the venous ulcer which affects 75% of the lesions of the lower limb; the diabetic foot that leads to amputation in a percentage of 15% of cases; decubitus ulcer caused by immobility and malnutrition; arterial vascular lesion caused mainly by arteriosclerosis and smoking which can degenerate into limb loss.

The knowledge of etiopathogenetic mechanisms and staging of lesions is a discriminating factor for designing an appropriate therapeutic pathway. The determination of the stage and state of the lesion must take place exclusively by using validated scales, when available.

Furthermore, in the local therapy of skin ulcers, the medication should not be considered a passive act or a simple covering of the wound but an instrument through which resolving phenomena are activated and obstacles to healing are removed. The main objective is that of promoting re-epithelialization, rather than cicatrisation, in order to obtain vital regenerated tissue.

Skin repair is a complex biological process that can be divided into four main phases:
▪ coagulation or haemostasis: formation of the fibrin plug and of an hypoxic underlying environment;
▪ inflammation: the activation of proteolytic enzymes in the affected area generates tissue degradation since they are able to consume part of the extracellular matrix. These lytic processes trigger inflammatory reactions that lead to vasodilation, diapedesis and arrival of neutrophils and macrophages at the site to phagocytize debris and pathogenic organisms and to release mediators such as cytokines that will recruit repairing cells;
▪ re-epithelialization and granulation: as inflammatory cells diminish, the main role in this phase is played by fibroblasts, endothelial cells and keratinocytes of the epidermis which, once recruited, synthesize growth factors. The loss of tissue is momentarily filled by a temporary matrix: fibrin and fibronectin, until the fibroblasts create new collagen, elastin and other molecules that form new extracellular scaffolding;
▪ tissue remodelling: this process is regulated by collagenase and other types of proteases that help maintain the balance between proliferation and degradation of collagen fibres and extracellular matrix.

A wound can become chronic when this ordered chronological sequence is subverted or even stopped.

In recent years, topical therapy of chronic skin lesions has focused on the use of potential bio-inductors, i.e. substances that, in contact with the wound bed, are able to release macromolecules of extracellular matrix and/or indirectly induce the recruitment of cellular populations which are essential for the formation of new vital tissue. With the aim of identifying molecules that meet these criteria, the researchers focused on the study of some proteins, including lactoferrin.

Lactoferrin is an 80 kDa glycoprotein capable of binding two Fe²⁺ or Fe³⁺ ions. It is the second most abundant protein in milk, after caseins. A wide range of functions has been described for lactoferrin. In particular, the protein has beneficial effects in the treatment of various infectious diseases caused by bacteria, fungi, protozoa and viruses. Furthermore, it is also known for important antioxidant, anti-neoplastic and anti-inflammatory properties and for the regulation of immune responses. Numerous formulations based on lactoferrin in the form of tablets or capsules are present on the market mainly as immune enhancers. Formulations for topical use have also been developed that can help repair skin tissues by intervening at different phases of the process.

It has been shown on healthy and diabetic mice that lactoferrin is able to speed up lesion closure since it can promote the production of cytokines and the infiltration of immune cells in the inflammation phase. At the same time, thanks to its immunomodulating properties, it is able to control an overabundant inflammatory response. As shown also *in vitro,* lactoferrin also intervenes in the granulation phase by promoting the balance of fibroblasts between synthesis of macromolecule and collagen degradation. In particular, the protein promotes the production of hyaluronic acid probably by induction of HAS2 expression (*hyaluronan synthase 2*)*.* Furthermore, it induces a significant increase in the rate of migration and invasion precisely of fibroblasts and keratinocytes. Some of the molecular mechanisms underlying the observed biological phenomena are described in the literature and relates to the interaction of this protein with the LRP-1 receptor (*low-density lipoprotein receptor* - *related protein-1*) and the consequent activation of ERK/MAPK-mediated intracellular signalling pathways. Among the downstream effects that have been demonstrated, phosphorylation of the Ser19 residue of the myosin light chain in human fibroblasts, underlying the increase in cell migration rate and collagen contraction, is included.

The use of lactoferrin in the treatment of skin lesions, also thanks to its antimicrobial effect, has aroused a strong interest as demonstrated by patents of gel-based formulations for topical use for tissue regeneration [WO2004024180A1; WO2012153301A1; US5834424A; US8545927B2; US20150320839A1].

The sodium mesoglycan is a preparation of natural glycosaminoglycans extracted from the intestinal mucosa of swine and is composed of heparan-sulphate (HS) (47.5%), dermatan-sulfate (DS) (35.5%), heparin (HEP) (8.5%) and chondroitin sulfate (CS) (8.5%). This mixture is a powder consisting of HEP (40% at low molecular weight, from 6500 to 10500 Da and 60% less than 12000 Da, with sulfonation grade 2.2-2.6), HS, considered as non-fractionated HEP from 12000 to 18000 Da up to 40000 Da and sulfonation degree 2.6, DS, deriving from the epimerization of the glucuronic acid of the CS (with molecular weight of 18000-30000 Da and degree of sulfonation 1.3). The methods for the preparation of mesoglycan, which can be extended to different mixtures of glycosaminoglycans, are reported in the patent US4987222A. It is commercially available in some European countries, both in the parenteral and oral form, for the treatment of vascular diseases with associated thrombotic risk such as deep vein thrombosis and chronic venous insufficiency. In fact, mesoglycan sodium can have an anti-atherogenic effect by inhibition of platelet adhesion, lipase stimulation, antithrombotic effects with anti-thrombin III activation and heparin II cofactor and pro-fibrinolytic action (stimulation of the activator of tissue plasminogen). These effects are due not only to the presence of HEP, but also to HS and DS, fundamental constituents of the vascular wall. Not by chance, mesoglycan is also able to exert an anti-oedematous action, restoring the physiological properties and electronegativity of selective capillary endothelial barriers.

Much of the information on mesoglycan comes from clinical studies. The few scientific *in vitro* works have demonstrated that mesoglycan is able to enhance the mitogenic activity of FGF (*Fibroblasts Growth Factor*) and to preserve it from denaturation and it inhibits the proliferation of smooth muscle cells in the G0/G1 phase and inhibition of protein synthesis. Its ability to protect growth factors such as FGFs and TGFs (*Transforming Growth Factor*) from tryptic degradation represents an important feature for its use for the treatment of lesions of the digestive tract [US5852004A].

It emerged from a study conducted on patients suffering from chronic venous insufficiency, and therefore subject to frequent ulcerations of the lower limbs that the treatment with mesoglycan reduces the estimated time for healing to 90 days compared to 136 days of the placebo in 75% of patients. Furthermore, the percentage of healed patients is 97.1% if treated with mesoglycan compared to 81.8% of the placebo. In this study, the authors also noted the possible side effects that affected 8% of the patients treated with mesoglycan and 7% with placebo [Arosio et al, 2001 Eur J Vasc Endovasc Surg. 22(4):365-72].

In particular, mesoglycan is able to strongly increase the processes of migration and cellular invasion thanks to a notable reorganization of the cytoskeleton in dermal fibroblasts, in epidermal keratinocytes and in human endothelial cells. Furthermore, this mixture of glycosaminoglycans enhances angiogenesis and shows an anti-inflammatory effect [Belvedere et al, 2017, Int J Mol Sci. 25;18(8). pii: E1614; Belvedere et al, 2017 Cell Adh Migr. 10:1-16].

It is therefore evident the continuous need to identify new and alternative effective treatments for the treatment of skin lesions.

### SUMMARY OF THE INVENTION

The present invention solves the aforementioned problems by means of a pharmaceutical or cosmetic composition comprising lactoferrin and mesoglycan.

It was surprisingly discovered that the association offers a synergistic effect on some cellular processes such as migration, invasion, proliferation, angiogenesis, inhibition of bacterial biofilms. The mesoglycan and lactoferrin association, object of the present invention, enhances the molecular variations both in terms of expression modulation and protein localization, with respect to the compounds administered individually. The association object of the invention tends to promote the main aspects of all the phases involved in skin regeneration, thus acting transversely. The composition according to the invention is therefore useful as a cosmetic or a medicament in particular for use in the treatment of skin lesions.

### DETAILED DESCRIPTION OF THE INVENTION

The term "lactoferrin", also known as lactotransferrin, defines a multifunctional globular protein bound or not to one or two iron atoms. Therefore, both the apo-lactoferrin when iron-free, the monoferric lactoferrin when bound to only one iron atom, and the ololactoferrin, when bound to two ferric ions are included in this term. It may be of human, bovine or mouse origin.

The term "mesoglycan" defines a mixture of glycosaminoglycans of animal origin, comprising heparan-sulphate (HS), heparin (HEP), dermatan-sulfate (DS) and chondroitin-sulphate (CS) in different percentages and with a different degree of sulforlation and length. It is generally found in the salified form of sodium mesoglycan but other possible forms are also included in the definition.

The term "skin lesions" defines any abnormality that occurs on the skin, from a small excoriation to a deep wound. These lesions can be primary or secondary, since an evolution of primary lesions. Primary lesions include burns, sunburns, insect bites, friction, viral infections (e.g. *Herpes Zoster*)*,* excoriations. Frequent types of lesions that can extend into depth and become chronic are ulcers. They comprise: venous ulcers, arterial ulcers, arteriovenous (or mixed) ulcers, diabetic ulcers (ischemic, neuropathic and mixed), pressure ulcers, vasculitic ulcers, neuropathic ulcers not related to diabetes, pressure or decubitus ulcers, ulcers in the course of hematological diseases, ulcers from metabolic and endocrine diseases: porphyria cutanea tarda, gout, cutaneous amyloidosis, cutaneous calcinosis, pretibial myxedema, hyper-thyroidism, hyperparathyroidism, homocystinuria, skin ulcers in obesity, ulcers in the course of infectious diseases (generalized and localized), ulcers in the course of skin diseases.

The object of the invention is intended to be usable and active in all the phases of skin regeneration described above.

The composition of the present invention can be formulated in a form suitable for oral administration or in a form suitable for parenteral or topical administration.

In a preferred embodiment of the present invention, said oral form is selected from tablet, capsule, solution, suspension, granules, oily pearl. In a further preferred embodiment of the present invention, said topical form is selected from cream, ointment, pomade, solution, suspension, eye drops, ovule, aerosol, spray, powder or gel. Attention will be paid to the formulation of bandages or gauzes or foams made with polymeric material comprising but not limited to alginates, chitosan, polyurethane, carbomer, differently functionalized cellulose, silicone, collagen, activated carbon, kaolin. In another preferred embodiment of the present invention, said parenteral form is selected from aqueous buffer solution or oily suspension. Said parenteral administration includes intramuscular, intravenous, intradermal, subcutaneous, intraperitoneal, intranodal, intrasplenic administration.

In the composition of the invention, preferably, the two active ingredients lactoferrin and mesoglycan are contained in a concentration range ranging from 0.1 to 100000 µg/ml for each active ingredient.

The composition according to the invention can furthermore and preferably comprise other types of substances of plant, animal or synthetic nature which will have to be considered included in the invention itself. The repertoire of these ingredients should be extended from the cosmetic to the pharmaceutical field, which ingredients are commonly used in skin care or in dermatology field. The example functional classes include, but are not limited to, abrasive, absorbent and adsorbent powder, pH buffer solutions, anaesthetics, painkillers, analgesics, anti-cellulite, anti-cracking, anti-irritant, anti-dandruff, antibacterial, antimicrobial, fungicidal, antifoaming and foaming agents, antioxidant, antiperspirant, antipruritic, antiseptic, antistatic, astringent agents, barrier, binders, sedatives, chelators, blood and lymphatic circulation stimulants, detergents, emulsifiers and co-emulsifiers, emollients, release controllers, denaturing agents, dispersants, gelling, cooling agents, fibres, essential oils and balms, perfumes and aromas, enzymes, cosolvents, cationic, anionic or amphoteric surfactants, colourings and colour stabilizers, alcohols and esters, fixatives, aromas, dyes, humectants, hydrophobic, hydrophilic agents and/or intermediates, lubricants, moisturizers, anti-masking, neutralizing agents, base ointments, pigments, plasticizers, polishing, sequestering agents, preservatives, propellants, opacifiers, lighteners, resins, regenerators, scrubs, soothing agents, equipped with sun protection filters or sun creams, thickeners, whiteners, viscosity stabilizers, vitamins, waxes, waterproofing and/or permeabilizing agents, accelerators of wound healing.

The composition according to the invention can preferably be administered to the patient who needs it in an amount, dose, frequency and duration of the treatment, adaptable according to the disorder from which the patient suffers, sufficient to cause the decrease of one or more symptoms associated with the disorder itself. Treatment will preferably have a frequency of administration every day, twice a day, three times a day, once a week, twice a week or three times a week. Preferably the treatment will have a treatment duration of a minimum of one or two weeks to a maximum limit at the discretion of the administering party.

The present invention can be better understood in the light of the following embodiment examples.

### BRIEF DESCRIPTION OF THE DRAWINGS

Fig. 1 - *in vitro* cell migration assay
Fig. 2 - *in vitro* cell invasion assay
Fig. 3 - cell proliferation assay: haemocytometer counting on BJ cells
Fig. 4 - assay of formation of capillary-like structures (*in vitro* angiogenesis)
Fig. 5 - inhibition of bacterial biofilm formation of *P. aeruginosa*
Fig. 6 - immunofluorescence characterization of the main protein markers on HaCaT cells
Fig. 7 - immunofluorescence characterization of the main protein markers on HUVEC cells
Fig. 8 - immunofluorescence characterization of the main protein markers on BJ cells

### EXPERIMENTAL PART

### Techniques and materials used

The lactoferrin used in this study was bought from the company Sigma Aldrich, a Merck KGaA group, but can be extended to all possible origins. The mesoglycan used in this study was bought by the company LDO (Laboratori Derivati Organici spa) but can be extended to any origin of the aforesaid compound.

The experiments were conducted *in vitro* on the following human cell lines: immortalized keratinocytes of dermis, HaCaT; immortalized fibroblasts of epidermis, BJ; primary endothelial cells deriving from the umbilical cord vein, HUVEC (*Human Umbilical Vein Endothelial Cell*). These cell lines were cultivated following the manufacturer's guidelines and kept sterile at 37°C, in a humidified atmosphere with 5% v/v of CO₂. The HUVECs were used for the experiments up to a step 10. Human lactoferrin was resuspended first in sterile deionized water and then diluted in PBS 1x (*Phosphate Buffered Saline*) or in the cell culture medium. The sodium mesoglycan was solubilized either in PBS 1x or in the cell culture medium before being administered. The two substances were administered both together and individually.

### Example 1: Cell migration analysis (Wound healing assay)

The HaCaT, BJ and HUVEC cells were seeded in 12 well multi-well plates in a number of 5 × 10⁵, 10 × 10⁵ and 3 × 10⁵, respectively, per well. After 24 hours the cells reached 100% confluence and a scratch was created in the middle of the well using a sterile p10 tip. After removing the growth medium and washing the wells twice with a 1x PBS solution, the cells were incubated in the presence of mesoglycan and lactoferrin at the reported concentrations by crossing the concentrations of 100 µg/ml of the first and 200 µg/ml of the second. An untreated control point was maintained for each cell line. All the experimental points were treated with mitomycin C at a concentration of 10 µg/ml to ensure that the mitosis was blocked. The plates containing the cells were then incubated in a chamber that was humidified and balanced at 5% v/v of CO₂ of an *Integrated Live Cell Workstation Leica AF-6000 LX* microscope. A 10x phase contrast objective was used to capture the images of the cells at a frequency of 10 minutes for at least 10 different positions of each experimental point. The migration rate was measured on individual cells from time 0 to 24 hours using Leica ASF software. At least 10 cells were selected and analysed for each photographed position.

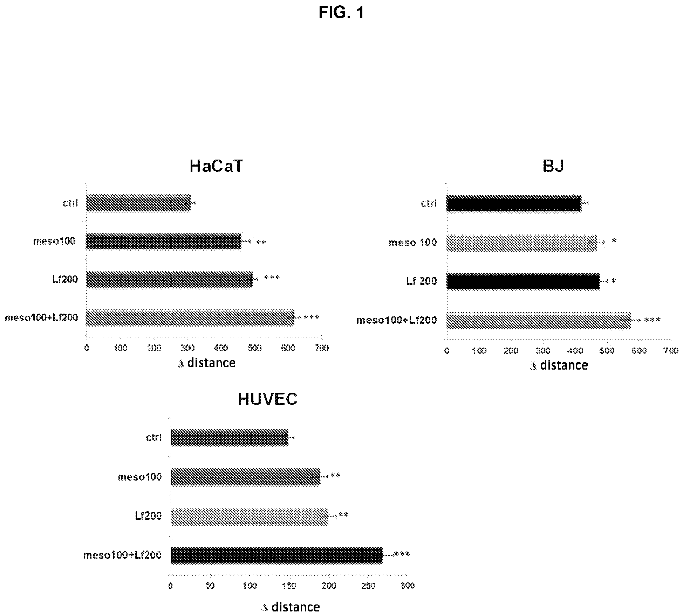

The HaCaT, BJ and HUVEC cells significantly increase their migration rate when treated with mesoglycan and lactoferrin individually, but this effect is greatly enhanced when the two substances are co-administered (Fig. 1). In detail, the percentage increase compared to the untreated control is expressed as:
for HaCaT cells

| meso100 | Lf200 | meso100+Lf200 |
|---|---|---|
| 23,3 | 32,8 | 60,9 |

for BJ cells

| meso100 | Lf200 | meso100+Lf200 |
|---|---|---|
| 11,4 | 11,8 | 29,2 |

for HUVEC cells

| meso100 | Lf200 | meso100+Lf200 |
|---|---|---|
| 27 | 31,1 | 69,6 |

### Example 2: Analysis of cellular invasion (Invasion assay)

The cells were plated on inserts with 8 µm pores, with a total diameter of 12 mm. A solution of *matrigel* diluted in the culture medium (1:3) of each cell line without growth factors and kept at 37°C until its complete gelation was previously seeded on these inserts. *Matrigel* is a mixture of proteins able to mimic the extracellular matrix. The HaCaT, BJ and HUVEC cells were seeded in a number of 5 × 10⁴ in the upper chamber of the insert in 350 µl of growth medium without serum (thus without growth factors with chemo-attracting properties). Instead, the treatments in 1.4 ml of complete growth medium were performed in the lower chamber. The cells were incubated for 24 hours at 37°C, in a sterile humidified atmosphere at 5% v/v of CO₂. At the end of the experimental time, the medium was aspirated from each insert, they were washed twice with PBS 1x, the cells present were then fixed in 4% paraformaldehyde in PBS for 10 minutes, permeabilized with methanol 100% for 20 minutes and stained with a violet *crystal* solution at 0.5% w/v for 15 minutes. Finally, the inserts were washed twice with PBS 1x, cleaned with a cotton swab to remove excess cells that were not inside the layer of *matrigel.* All the experimental points were treated with mitomycin C 10 µg/ml to block mitoses. The number of cells within the layer was counted by images captured in at least 12 random fields on the insert under the EVOS microscope, 10x objective.

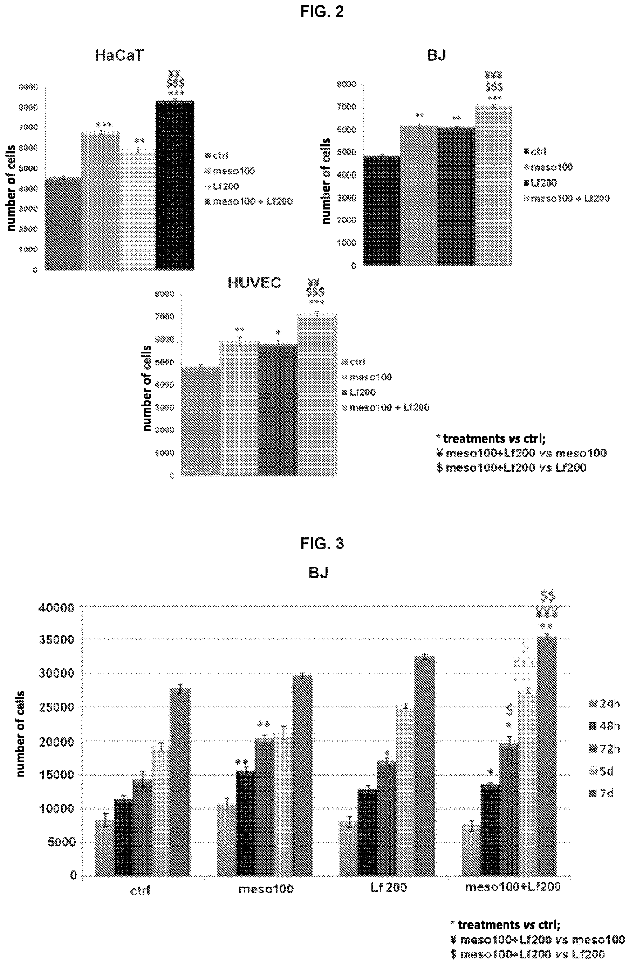

The number of HaCaT, BJ and HUVEC cells that are able to invade the layer of *matrigel* significantly increases in the presence of mesoglycan and lactoferrin alone (Fig.2). This effect is further increased when the two substances are present together. In detail, the increase compared to the untreated control in percentage terms is quantified as follows:
for HaCaT cells

| meso100 | Lf200 | meso100+Lf200 |
|---|---|---|
| 49,7 | 30,5 | 75,7 |

for BJ cells

| meso100 | Lf200 | meso100+Lf200 |
|---|---|---|
| 27,6 | 26 | 46,2 |

for HUVEC cells

| meso100 | Lf200 | meso100+Lf200 |
|---|---|---|
| 30,7 | 28,1 | 47,6 |

### Example 3: Haemocytometer counting

The BJ cells were plated in a number of 2 × 10⁴ in a 12 well multi-well plate. After 12 hours the growth medium was replaced by a serum-free medium so as to synchronize the cell cycle for all cells. The cells were harvested at 24, 48, 72 hours, 5 and 7 days starting from the treatments performed with the mesoglycan and lactoferrin association by crossing the concentrations of 100 µg/ml of the first and 200 µg/ml of the second. Equal volumes of the stain *trypan blue,* 0.4% w/v of cell suspension were mixed. About 10 µl of this mixture was placed on the edge of a slide in the Burker chamber. The haemocytometer counting chamber was visualized with an optical microscope (10x objective) and the mean of the number of cells arranged on 5 of the 9 quadrants displayed was performed to calculate the live cells per ml of suspension. This value was multiplied by the dilution factor 2 and by 10⁴. The mesoglycan and lactoferrin association, in addition to the single substances, induces the increase of cell proliferation starting from 48 hours of administration (Fig. 3). In detail, compared to the untreated control, the changes in the number of cells in percentage terms are:

| | meso100 | Lf200 | meso100+Lf200 |
|---|---|---|---|
| 24h | 28 | -3,2 | -4,8 |
| 48h | 36,5 | 10,3 | 18,8 |
| 72h | 42 | 27,4 | 36,4 |
| 5d | 4,9 | 33,1 | 39,2 |
| 7d | 3,4 | 22,2 | 35,4 |

### Example 4: In vitro angiogenesis (Tube formation assay)

The wells of a 24 well multi-well plate were coated with a layer of *matrigel* in a 1:1 solution with the culture medium of HUVEC cells on ice. The plate was kept in an incubator at 37°C until gelation. The HUVEC cells were seeded on the gel in a number of 5 × 10⁴ per well in the presence or not of the selected treatments. The cells were incubated under sterility at 37°C, in a humidified atmosphere with 5% v/v of CO₂ overnight. After about 12 hours, images (10x objective) were taken with the EVOS microscope and the length of each tube and the number of branches were calculated using the Angiogenesis Analyzer software for ImageJ.

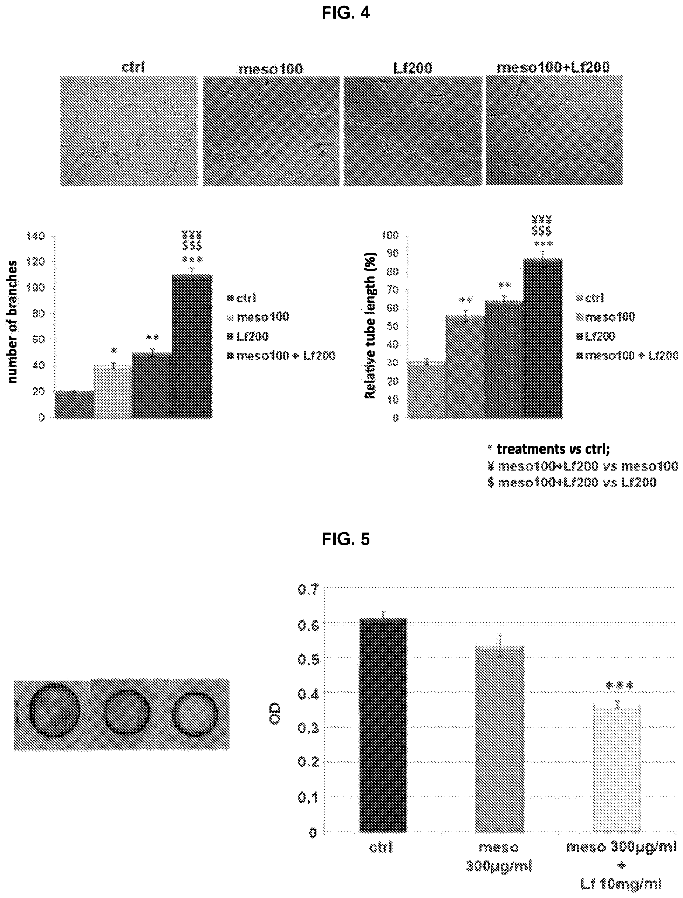

The mesoglycan and lactoferrin association induces the increase in the ability of HUVEC cells to constitute capillary-like structures in 12 hours. It is possible to note from Fig. 4 that the number of branching points and the length of the tubes increase significantly not only with the single treatments but above all when they are present together.

### Example 5: Analysis of the effect on bacterial biofilm formation

In order to determine the ability of the mesoglycan and lactoferrin association to inhibit the formation of *Pseudomonas aeruginosa* biofilm, a colony of *P. aeruginosa* PAO1 was inoculated in 7 ml of *Mueller Hinton Broth* (MHB) and incubated *overnight* at 37°C under stirring. Following a spectrophotometric reading at 600 nm of the *overnight* culture, a culture of 1 × 10⁶ cells/ml in *Luria-Bertani* (LB) *Broth* was prepared and supplemented with 2% glucose. This culture was used to fill the wells of a 96 well plate (100 µl/well). A well with only bacterial cells was used as a positive control for biofilm formation. In two separate wells, mesoglycan alone (300µg/ml) and mesoglycan combination 300µg/ml and lactoferrin 10mg/ml were added in separate wells and incubated *overnight* at 37°C without stirring (static culture). Subsequently, the culture medium was removed from each well, and the not adherent cells of *P. aeruginosa* were removed by washing the wells with PBS. After this washing, the formed biofilm mass was fixed by incubating the plate at 60°C for 1 hour, and subsequently highlighted with the violet *crystal* staining. The biofilms were briefly stained with 3% v/v CV for 10 min. The excess violet *crystal* was removed with water, while that trapped in the biofilm was recovered by adding 200 µl/ml of 70% ethanol to each well and subjected to spectrophotometric reading (OD600nm).

The action of lactoferrin is significant compared to the lack of ability of the mesoglycan to act as an anti-microbial.

### Statistical analyses

Statistical analyses were performed on resultants deriving from at least three experiments with similar results in which the experimental points were maintained in technical triplicate. Statistical comparisons were performed by means of tow-tailed *Student's* t-test with different variance. The differences were considered significant if in case of *p value* at least <0.05 * / ¥ / $ ; <0.01 ** / YY / $$; <0.001 *** / ¥¥¥ / $$$.

### Example 6-8: Immunofluorescence (analysis by confocal microscopy)

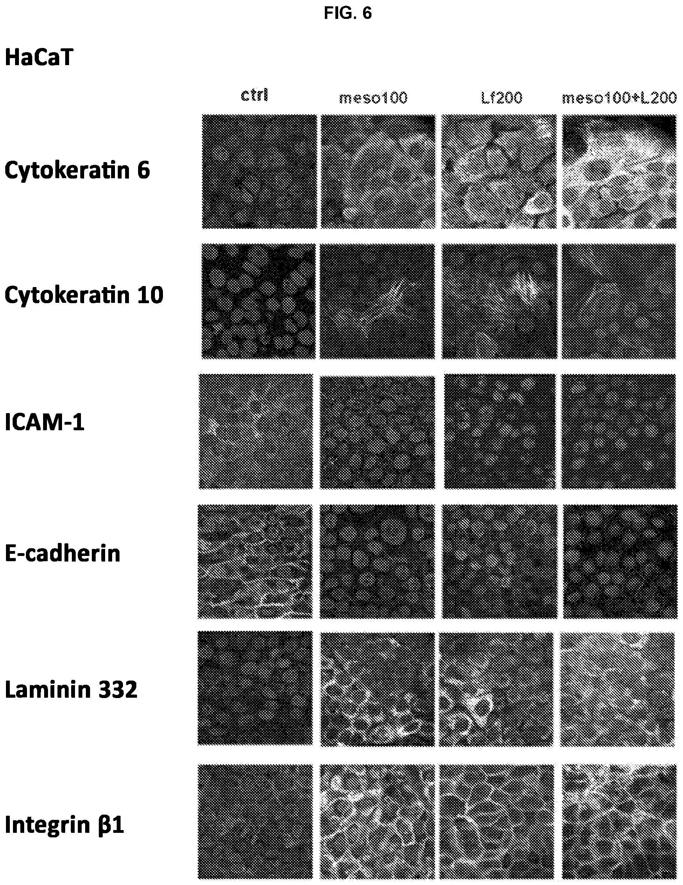

The HaCaT, BJ and HUVEC cells were plated in a number of 2 × 10⁴ on coverslips 12 mm in diameter and 0.1 mm thick. After 24 hours the treatments with mesoglycan and lactoferrin were performed for 48 hours, by crossing the concentrations of 100 µg/ml of the first and 200 µg/ml of the second. At the end of the experimental time, the cells were fixed with a 4% paraformaldehyde solution in PBS 1x for 5 minutes, permeabilized with 0.5% v/v triton in PBS 1x for more 5 minutes and incubated with goat serum (20 % v/v in PBS) for 30 minutes. Subsequently, the cells were incubated overnight at 4°C with antibodies against VEGF (*Vascular Endothelial Growth Factor*) 1:100, CD31 (*cluster of differentiation* 31) 1:100, ICAM-1 *(Intercellular Adhesion Molecule-1)* 1:100, N-cadherin 1:250, laminin 332 1:250, fibronectin 1:250, integrin β1 1:250, cytokeratin 6 1:500, cytokeratin 10 1:500, E-cadherin 1:1000, vimentin 1:1000, α-SMA (*alpha-Smooth Muscle Actin*) 1 :250, FAP-α (*Fibroblast activation protein-alpha*) 1:250, vinculin 1:100. On the next day the slides with the cells were washed and incubated for 2 hours at room temperature in the dark in the presence of secondary anti-mouse, anti-rabbit and anti-goat fluorescent antibodies conjugated with fluorochromes 488 and 555. To stain the nuclei, fluorescent Hoechst stain 33342 1:1000 was added to the solution with the secondary antibodies. Finally, FITC 1:500 fluorochrome-conjugated phalloidin was used for 30 minutes in the dark at room temperature in order to highlight the polymerization of F-actin. Subsequently the slides were mounted on coverslips with a transparent mounting solution and kept at 4°C until confocal analysis. The emission signals were analysed with laser 488 nm Ar (argon) and 555 nm He-Ne (helium-neon). The samples were scanned vertically from the first to the last focal plane for a total distance of 5µm to a 63x immersion objective. The images (Fig. 6-8) were generated using the Zeiss ZEN Confocal software and processed with ImageJ, Adobe Photoshop and Microsoft Power Point software.

### Results

Lactoferrin and sodium mesoglycan show significant pro-migratory and pro-invasive effects. As evident in example 1, by means of the cell migration assay it was possible to demonstrate the increase in the distance travelled over 24 hours by all the three HaCaT, BJ and HUVEC cell lines when treated with mesoglycan 100 µg/ml and lactoferrin 200 µg/ml individually. When these two treatments are combined together the migration rate undergoes a considerable increase, which is significant compared not only to the untreated control but also to individual experimental points. Instead, the pro-invasive effect is described in Figure 2 whose histograms show the number of cells that crossed the layer of *matrigel* over 24 hours. In the presence of the aforesaid treatments, this number increases significantly. Also in this case the effect enhancement on the biological process under examination is evident in the case of the co-administration of mesoglycan and lactoferrin.

Figure 3 shows the histogram depicting the BJ human fibroblast count after 24, 48, 72 hours, 5 and 7 days of treatment with sodium mesoglycan 100 µg/ml with lactoferrin 200 µg/ml. The proliferation rate appears to be affected by the presence of mesoglycan and lactoferrin at different times. Therefore, both substances are active in allowing fibroblasts to proliferate more quickly both in the case of single treatments and co-administration. In addition to the processes of cell migration and invasion, proliferation is also a crucial event in the different phases of tissue regeneration in skin lesions. In fact granulation, a moment in which the fibroblasts intervene with collagen production, becomes much more effective if the number of such protagonist cells increases significantly. The result obtained on the BJ cell population represents a strength for the mesoglycan/lactoferrin association due to its ability to positively influence different aspects of epithelial repair.

As previously mentioned, another essential moment for the correct skin regeneration is the formation of new vascular structures that are able to bring oxygen, nutrients and cells to the damaged site. For this reason, an *in vitro* angiogenesis assay was also performed in the manner described above. Figure 4 shows the images of capillary-like structures that HUVEC cells were able to form in 12 hours from treatments. In the presence of sodium mesoglycan and lactoferrin, at their respective concentrations of 100 µg/ml and 200 µg/ml, these cells organize themselves to form not only longer capillary tubes but also with a greater number of branching points, compared to the untreated control point. It is possible to note that the treatment of lactoferrin and mesoglycan brings more pronounced pro-angiogenic properties.

In literature, the characteristics of lactoferrin as a protein inhibiting bacterial growth and biofilm formation are known, therefore the adhesion of bacterial populations. These anti-bacterial properties guarantee a protective effect of lactoferrin that supports the repair of skin lesions. For this reason, an evaluation assay of the biofilm formation of the *P. aeruginosa* PAO1 strain was performed. From the histogram shown in Figure 5 it is possible to notice that, compared to the positive control (the only cells of *Pseudomonas* where 100% of the possible biofilm has formed), the mesoglycan alone at 300 µg/ml inhibited 14% of the biofilm, therefore it remained at non-significant levels of efficacy. The experimental point with the association between mesoglycan 300 µg/ml and lactoferrin 10 mg/ml, instead, inhibited the biofilm of the Gram-negative bacterium by more than 40%. In this way, among the effects of the mesoglycan/lactoferrin association it was possible to include also an antimicrobial effect which contributes to a better and faster tissue repair in skin lesions. In particular, mesoglycan alone is not able to inhibit bacterial adhesion unlike lactoferrin, which brings about these characteristics in case of co-administration.

The characterization of the effect of mesoglycan and lactoferrin as single agents but in particular in association was also performed by immunofluorescence assays with which some of the main molecular markers involved in the activation of the three HaCaT, BJ and HUVEC cell lines were evaluated.

Considering each cell line, Figure 6 shows some proteins characterizing the biological processes previously examined on human HaCaT keratinocytes. First, cellular differentiation was demonstrated by the increase in the expression of the two cytokeratins 6 and 10. Differentiation represents an important moment for the final phases of re-epithelialization. In fact, these cells pass from a basal layer to the corneum stratum and are able not only to maintain a correct epidermal barrier but also to form it following a lesion. Some important aspects concerning pro-migratory activation are based on the significant organization of the plasma membrane of integrin β1, the dramatic reduction in E-cadherin expression and the cytoplasmic diffusion of laminin 332. In parallel, the decrease in ICAM-1 expression in keratinocytes can be linked not only to differentiation (basal keratinocytes have greater expression than this protein) but also to the acquisition a greater capacity of these cells to resist inflammatory stimuli and external insults.

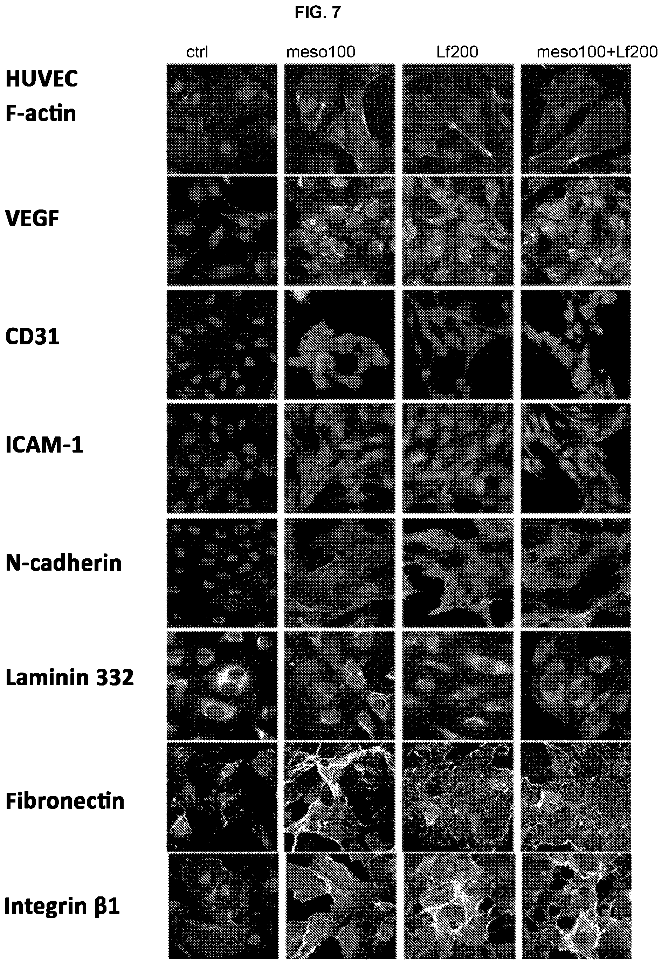

The panels shown in Fig. 7 show that on HUVEC cells the association induces the endothelium-mesenchymal transition, i.e. it allows the passage from a "resident" phenotype to one that is able to migrate and invade, aimed therefore at the formation of capillaries. As confirmation of the transition, we analysed fibronectin, N-cadherin, integrin β1, ICAM-1 demonstrating a strong increase in expression for all four of these proteins. On the other hand, the actin cytoskeleton appears strongly organized and laminin 332 diffuses from the perinuclear area to the cell membrane, ready to be secreted and contribute to the formation of the extracellular matrix.

Finally, the increase in VEGF and CD31 expression further demonstrates angiogenesis induction.

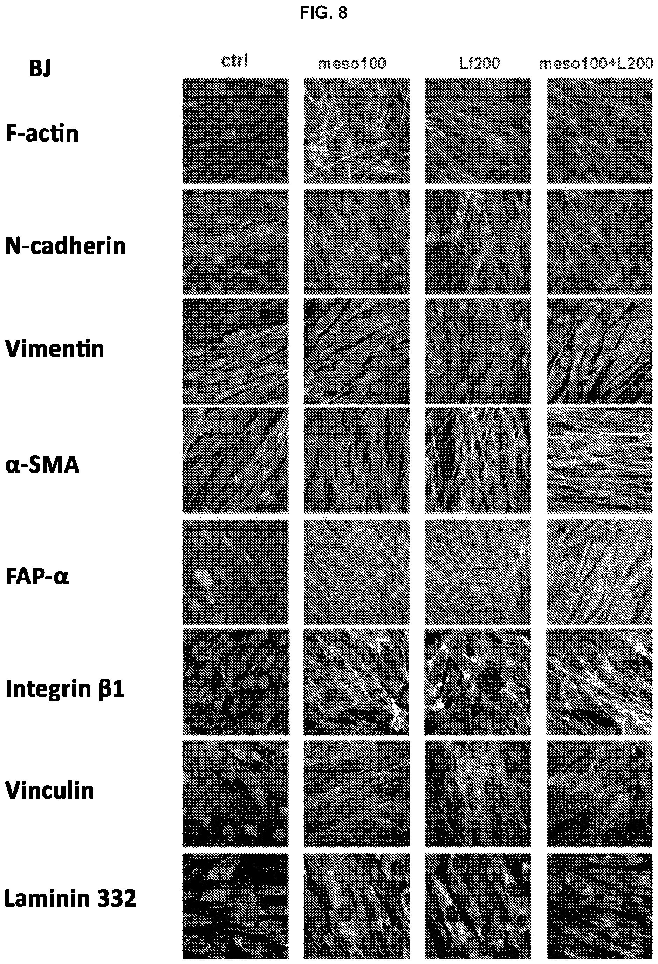

Fig. 8 shows the fluorescences performed on human BJ fibroblasts. It was possible to demonstrate their activation after 48 hours of treatment with mesoglycan and lactoferrin but more markedly with the association of the two thanks to the strong organization of the F-actin cytoskeleton, of vimentin, of N-cadherin and of α-SMA. Other markers of fibroblast maturation are FAP-α, integrin β1 and vinculin whose expression increases and laminin 332 whose localization passes from the perinuclear zone towards the plasma membrane.

In all the cases examined, the mesoglycan and lactoferrin association enhances the molecular variations both in terms of expression modulation and protein localization, compared to the compounds administered individually. These aspects highlight the inventive activity of the subject invention. The way in which the mesoglycan and the lactoferrin intervene with different effects in a more or less marked manner, depending on the cellular process examined, makes it possible for the invention to fall within various types of medications for skin regeneration. Examples of these medications are those able to promote (biochemical and/or physicochemical) autolysis and the removal of part of the material (*debridement*)*,* those granulating, anti-microbial and eudermic. Each of these types are able to intervene in distinct phases of tissue repair. The tested association, instead, tends to promote the main aspects of all these phases, thus acting transversely.

## Claims

1. A pharmaceutical or cosmetic composition comprising lactoferrin and mesoglycan.

2. The composition according to claim 1 wherein lactoferrin and mesoglycan are each contained in a concentration range ranging from 0.1 to 100000 µg/ml.

3. The composition according to claim 1 or 2 **characterized by** the fact of being formulated in a form suitable for oral administration.

4. The composition according to claim 3 wherein said form of oral administration is selected from tablet, capsule, solution, suspension, granules or oily pearl.

5. The composition according to claim 1 or 2 **characterized by** the fact of being formulated in a form suitable for parenteral administration.

6. The composition according to claim 5 wherein said parenteral administration form is selected in the group consisting of aqueous buffer solution and oily suspension.

7. The composition according to claim 5 or 6 wherein said parenteral administration is selected in the group consisting of intramuscular, intravenous, intradermal, subcutaneous, intraperitoneal, intranodal or intrasplenic administration.

8. The composition according to claim 1 or 2 **characterized by** the fact of being formulated in a form suitable for topical administration.

9. The composition according to claim 8 wherein said form of topical administration is selected in the group consisting of cream, pomade, ointment, unguent, gel, bandage and gauze.

10. The pharmaceutical composition according to any of the claims 1-9 for use in the treatment of skin lesions selected in the group consisting of burns, sunburns, insect bites, frictions, from viral infections, excoriations, ulcers.

11. The pharmaceutical composition for use according to claim 10 where said ulcers are selected in the group consisting of venous ulcers, arterial ulcers, arteriovenous or mixed ulcers, ischemic, neuropathic and mixed diabetic ulcers, pressure ulcers, vasculitic ulcers, neuropathic ulcers not related to diabetes, pressure or decubitus ulcers, ulcers in the course of haematological diseases, ulcers in obesity, ulcers in the course of infectious diseases (generalized and localized), ulcers in the course of skin diseases.

12. A non-therapeutic cosmetic use of the cosmetic composition according to any one of claims 1-9.

## Patentansprüche

1. Pharmazeutische oder kosmetische Zusammensetzung, die Lactoferrin und Mesoglykan umfasst.

2. Zusammensetzung nach Anspruch 1, wobei Lactoferrin und Mesoglykan jeweils in einem Konzentrationsbereich von 0,1 bis 100.000 µg/ml enthalten sind.

3. Zusammensetzung nach Anspruch 1 oder 2, **dadurch gekennzeichnet, dass** sie in einer zur oralen Verabreichung geeigneten Form formuliert ist.

4. Zusammensetzung nach Anspruch 3, wobei die Form zur oralen Verabreichung aus Tablette, Kapsel, Lösung, Suspension, Granulat oder öliger Perle ausgewählt ist.

5. Zusammensetzung nach Anspruch 1 oder 2, **dadurch gekennzeichnet, dass** sie in einer zur parenteralen Verabreichung geeigneten Form formuliert ist.

6. Zusammensetzung nach Anspruch 5, wobei die parenterale Verabreichungsform aus der Gruppe ausgewählt ist, die aus wässriger Pufferlösung und öliger Suspension besteht.

7. Zusammensetzung nach Anspruch 5 oder 6, wobei die parenterale Verabreichung aus der Gruppe ausgewählt ist, die aus intramuskulärer, intravenöser, intradermaler, subkutaner, intraperitonealer, intranodaler oder intrasplenischer Verabreichung besteht.

8. Zusammensetzung nach Anspruch 1 oder 2, **dadurch gekennzeichnet, dass** sie in einer zur topischen Verabreichung geeigneten Form formuliert ist.

9. Zusammensetzung nach Anspruch 8, wobei die Form zur topischen Verabreichung aus der Gruppe ausgewählt ist, die aus Creme, Pomade, Balsam, Salbe, Gel, Verband und Gaze besteht.

10. Pharmazeutische Zusammensetzung nach einem der Ansprüche 1-9 zur Verwendung bei der Behandlung von Hautläsionen, die aus der Gruppe ausgewählt sind, die aus Verbrennungen, Sonnenbränden, Insektenstichen, Schürfwunden, viralen Infektionen, wunden Stellen, Geschwüren besteht.

11. Pharmazeutische Zusammensetzung zur Verwendung nach Anspruch 10, wobei die Geschwüre aus der Gruppe ausgewählt sind, die aus venösen Geschwüren, arteriellen Geschwüren, arteriovenösen oder gemischten Geschwüren, ischämischen, neuropathischen und gemischten diabetischen Geschwüren, Druckgeschwüren, vaskulitischen Geschwüren, neuropathischen Geschwüren, die nicht im Zusammenhang mit Diabetes stehen, Druck- oder Dekubitalgeschwüren, Geschwüren im Rahmen von hämatologischen Erkrankungen, Geschwüren bei Adipositas, Geschwüren im Rahmen von Infektionskrankheiten (generalisiert und lokalisiert), Geschwüren im Rahmen von Hauterkrankungen besteht.

12. Nicht therapeutische kosmetische Verwendung der kosmetischen Zusammensetzung nach einem der Ansprüche 1-9.

## Revendications

1. Composition pharmaceutique ou cosmétique comprenant de la lactoferrine et du mésoglycane.

2. Composition selon la revendication 1, dans laquelle la lactoferrine et le mésoglycane sont chacun contenus dans une plage de concentration allant de 0,1 à 100000 µg/ml.

3. Composition selon la revendication 1 ou 2, **caractérisée par** le fait d'être formulée sous une forme adaptée à l'administration orale.

4. Composition selon la revendication 3, dans laquelle ladite forme d'administration orale est choisie parmi un comprimé, une capsule, une solution, une suspension, des granulés ou une perle huileuse.

5. Composition selon la revendication 1 ou 2, **caractérisée par** le fait d'être formulée sous une forme adaptée à l'administration parentérale.

6. Composition selon la revendication 5, dans laquelle ladite forme d'administration parentérale est choisie dans le groupe constitué par une solution tampon aqueuse et une suspension huileuse.

7. Composition selon la revendication 5 ou 6, dans laquelle ladite administration parentérale est choisie dans le groupe constitué par administration intramusculaire, intraveineuse, intradermique, sous-cutanée, intrapéritonéale, intranodale ou intrasplénique.

8. Composition selon la revendication 1 ou 2, **caractérisée par** le fait d'être formulée sous une forme adaptée à l'administration topique.

9. Composition selon la revendication 8, dans laquelle ladite forme d'administration topique est choisie dans le groupe constitué par la crème, la pommade, le baume, l'onguent, le gel, le bandage et la gaze.

10. Composition pharmaceutique selon l'une quelconque des revendications 1 à 9, destinée à être utilisée dans le traitement des lésions cutanées choisies dans le groupe constitué par les fesses, les coups de soleil, les piqûres d'insectes, les frictions, les infections virales, les excoriations, les ulcères.

11. Composition pharmaceutique à utiliser selon la revendication 10, dans laquelle lesdits ulcères sont choisis dans le groupe constitué par les ulcères veineux, les ulcères artériels, les ulcères artério-veineux ou mixtes, les ulcères diabétiques ischémiques, neuropathiques et mixtes, les escarres, les ulcères vasculitiques, les ulcères neuropathiques non liés au diabète, les escarres de décubitus, les ulcères au cours de maladies hématologiques, les ulcères d'obésité, les ulcères au cours de maladies infectieuses (généralisées et localisées), les ulcères au cours de maladies cutanées.

12. Utilisation cosmétique non thérapeutique de la composition cosmétique selon l'une quelconque des revendications 1 à 9.
